# EUROPEAN PATENT APPLICATION

(11) **EP 4 616 839 A1**
(43) Date of publication of application: **17.09.2025**
(21) Application number: 25162186.8
(22) Date of filing: 06.03.2025
(51) Int. Cl.: A61K 8/02, A61K 8/34, A61K 8/37, A61K 8/55, A61K 8/73, A61Q 1/12, A45D 40/00, A45D 33/18

(54) **MAKE-UP ARTICLE AND METHOD FOR THE PRODUCTION THEREOF**

(30) Priority: 11.03.2024 IT 202400005419
(71) Applicant: Chromavis S.p.A., 26010 Offanengo (CR) (IT)
(72) Inventor: Sangalli, Andrea Rubens, 26010 Campagnola Cremasca (CR) (IT); Gaboardi, Mauro, 26026 Pizzighettone (CR) (IT); Guizzetti, Giorgio, 20871 VIMERCATE (MB) (IT)
(74) Representative: Vanosi, Adelio Valeriano

(57) **Abstract**

A method for producing a make-up article (1) comprising the following steps:
a. preparing a non-make-up support (2);
b. preparing a frame (3) with a surface (4) formed of a screened area (S) and at least one passage area (P1, P2, P3) that determines the shape of a decoration, to be impressed onto the support, the passage area (P1, P2, P3) being formed of a plurality of mutually adjacent holes (F);
c.placing the frame (3) on the support (2);
d. pouring a make-up product (5) onto the frame;
e. spreading the make-up product (5) over the surface (4) so that the said product is deposited on the part of the support facing the passage area (P1, P2; P3);
f. removing the frame (3) from the support;
g. leaving the make-up product (5) to dry/desiccate on the support (2).

## Description

### FIELD OF THE INVENTION

The present invention relates to a make-up article and a method for the production thereof.

### BACKGROUND ART

Conventional cosmetics or make-up articles normally consist of a make-up product housed in a compartment within a container that contains the said products.

To create a multi-coloured palette, multiple containers are generally coupled together or colours are combined within the same container.

The compartment can be formed within a godet, generally made of aluminium, or in a plastic container, etc.

Commonly known articles are very conventional, but in the cosmetics field, there is an ongoing search for new offerings and highly innovative solutions that change or revolutionise the way a make-up article is marketed or used.

EP 2 425 741 A1 and ES 2 027 585 A6 describe known make up products.

### SUMMARY OF THE INVENTION

The object of the present invention is to provide a make-up article which is an improvement on the prior art.

A further object of the invention is to provide a make-up article that is totally revolutionary with respect to the commonly known ones in terms of functionality and results delivered.

This and other objects are achieved by means of a make-up article produced according to the technical teachings and the method set out in the claims annexed hereto.

Advantageously, the make-up article according to the present invention has a lower environmental impact with respect to conventional articles and, in some cases, at least some of the component parts can be reused.

### BRIEF DESCRIPTION OF THE FIGURES

Further features and advantages of the innovation will become clearer in the description of a preferred but not exclusive embodiment of the make-up article, illustrated - by way of a non-limiting example - in the drawings appended hereto, in which:
Figure 1 is a perspective view of some parts which are useful for making the article according to the present invention following the proposed method;
Figures 2 to 4 show some steps in the production of the present invention, also in a perspective view and in a simplified manner;
Figure 5 shows a make-up article produced according to the present invention, in a perspective view;
Figure 6 is an enlarged view of the detail shown in the square of Figure 1; and
Figure 7 is a schematic view of a possible application of the article in Figure 5.

### DETAILED DESCRIPTION OF THE INVENTION

With reference to the figures stated, reference number 1 is used to denote, as a whole, a make-up article.

The make-up article 1 can be any article such as, for example, an eye shadow, a blusher, a powder lipstick, a face highlighter, a bronzer, etc.

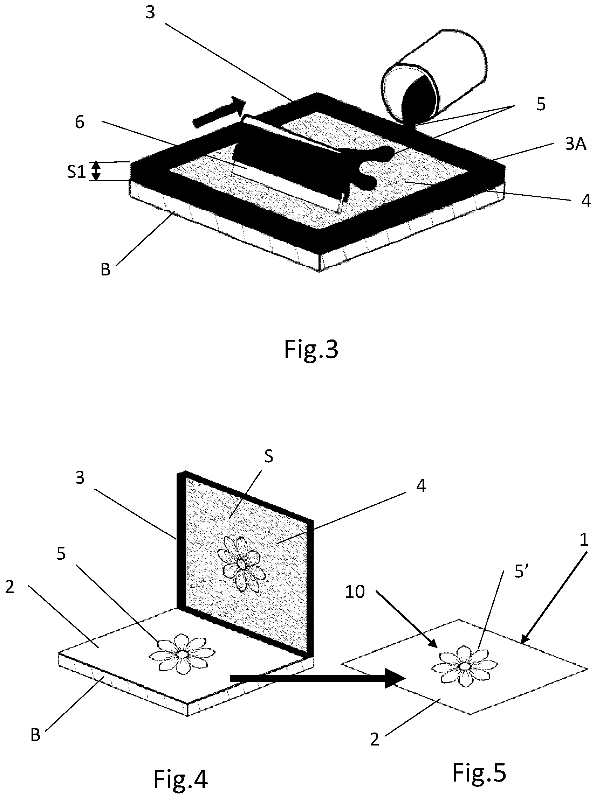

As can be seen in Figure 5, the said article comprises a support 2 (essentially laminar in form) on top of which there is a make-up product 5' featuring a decoration 10.

The support preferably has a thickness that is negligible with respect to the width and length thereof.

Advantageously, the thickness of the support 2 can be between 0.1 mm and 3 mm, preferably between 0.4 mm and 1.5 mm.

The make-up product essentially adheres to the support 2, but can be taken therefrom by a user (for example by running a finger or a moistened applicator thereover) to apply the make-up.

The use of the make-up article will be explained in more detail later.

Materials that can be used for the support 2 include paper, cardboard, natural fibre fabrics such as hemp or cotton, plastics such as PET and PP.

Preferably, the ideal support is made of paper (essentially a sheet of natural paper, which is therefore made only of cellulose, possibly recycled cellulose) which is as smooth as possible and has a weight of between 150 gsm and 300 gsm.

Cardboards with a higher weight can also be used; in this case the design may not include scorings and specific cuts.

Paper and cardboard weighing less than 150 gsm are less suitable for the production of the article 1 as they tend to deform in an uncontrolled manner during the article production process.

Indeed, as will be explained in more detail later, when the make-up product 5 is deposited on the support 2 during the production step, the said product contains a certain amount of solvent, which can impregnate and therefore deform the support 2.

In the present document, the "make-up product" will therefore be denoted by two different reference numbers.

Reference number 5 will be used to denote the make-up product with added solvent (as in Figure 3) and reference number 5' will denote the 'dry' make-up product (as in Figure 5), i.e. with the solvent at least partially evaporated in order to form the final make-up article 1.

Returning to the concept expressed above, to prevent deformation of the support during the production step, it is possible to use paper weighing less than 150 gsm, as long as the said paper is coated (typically with PP plastic or silicone).

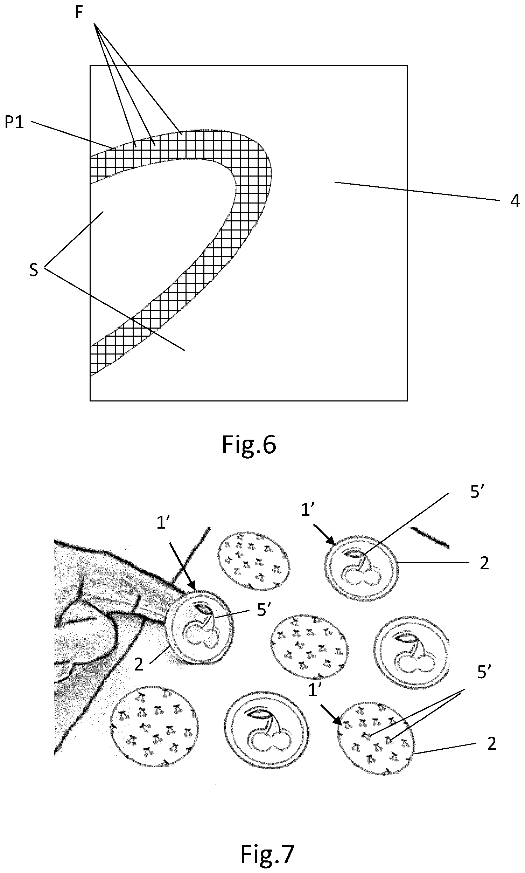

The possibility of using lighter paper, with plastic coatings, is also useful in order to create stickers (as shown in Figure 7) that can be used as support for the provision of product refills or customised palettes.

Finally, printing onto fabrics allows the creation of a make-up article which will have a second life through the said fabric, which can be reused (for example as a scarf, a handkerchief, etc.).

The make-up product forms a decoration on the support 2, which can be a detailed drawing, wording, a simple or complex geometric shape, etc.

The decoration 10 can also feature several different colours. In this case, there are several make-up products in different colours present on the support 2.

The make-up article 1 is produced with a method comprising the following steps, preferably in succession.

Step a) envisages the preparation of a non-make-up support 2.

The (non-make-up) support 2 can be, for example, a laminar support, i.e. formed of a sheet of material as specified above.

The term 'non-make-up' means that the said support is essentially inert and does not, per se, release a make-up substance if touched or moistened.

There then follows step b), wherein a frame 3 is prepared, the frame 3 having a surface 4 formed of a screened area S and at least one passage area P1, P2, P3, which determines the shape of a decoration 10 (or a part of the decoration, as will be seen later) to be impressed onto the support.

Each passage area P1, P2, P3 may be formed of a plurality of mutually adjacent holes F.

The holes F that determines each passage area P1, P2, P3 are arranged very closely together. More specifically, the distance between one hole and the adjacent hole (or holes) thereto can be between 0.2 and 2 mm, preferably between 0.2 and 0.8 mm (i.e. preferably equal to the diameter of the strands that form the surface 4).

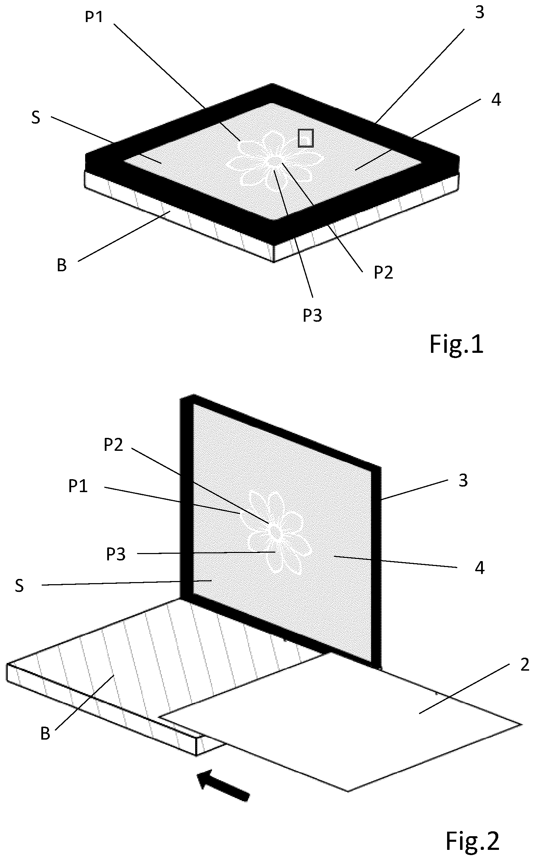

In the example shown in Figures 1 and 2 (and consequently also 5), the decoration essentially depicts a flower (a daisy).

There are therefore three passage areas.

The first passage area P1 determines the external perimeter of the flower and the petals at the same time.

The second passage area P2 determines the perimeter of the central disk (the yellow disk in the daisy).

The third passage area P3 determines the base of the petals close to the central disk.

Advantageously, each hole F (better visible in Figure 6) can have a surface area of between 0.01 and 0.12 mm², preferably between 0.015 and 0.12 mm².

Each hole F can have a quadrangular, preferably square, section in a plan view. This is clearly visible in Figure 6.

According to the invention, the surface 4 of the frame 3 is a fabric.

Therefore, each hole F is perimetrically delimited (or better determined) by the threads that form the warp and weft of the fabric.

The screened part S can be determined by the said fabric impregnated with a hardened photosensitive emulsion.

For the production of the frame, it is possible to start with a perimeter (for example, quadrangular) frame 3A with a certain thickness S1, made - for example - of wood, metal, or plastic.

A (pure, non-recycled) fabric is fastened, stretched taut, onto one side of the frame (for example the side facing the base B in Figure 2).

Advantageously, the fabric has a thread count (a unit of measurement that corresponds to the number of threads woven vertically and horizontally per square centimetre) that is between 55 and 24.

The threads/strands that form the fabric can be made of nylon, polyester, metal, etc.

The diameter of the threads can measure between 0.2 mm and 0.8 mm.

The fabric is soaked in a photosensitive emulsion, preferably of a water-soluble type, for example by immersion thereof in the said emulsion.

The whole assembly is then dried in hot air, at 35-40°C, away from light, for approximately 20 minutes.

Then a positive reproduction of the decoration 10 on a transparent sheet (for example a transparency) is placed on top of the pre-soaked fabric.

The whole assembly is then irradiated with an appropriate light.

In the parts where the transparency is transparent, the light reaches and therefore hardens the emulsion.

In the parts where the transparency is printed with the decoration 10, the light does not reach the emulsion (since the light is absorbed by the ink); therefore, this part of the emulsion does not harden.

Alternatively, the photosensitive emulsion can be hardened directly in the screened area (therefore, without transparencies) through the use of computer-controlled UV light projection systems.

Later, the unhardened part of the emulsion is removed by washing, for example with water; this dissolves the said emulsion thereby clearing the passage(s) P1, P2, P3.

At this point, the frame 3 is dried and so is ready to be used.

In Step c) the frame 3 having the surface 4, is placed above the support 2, with the fabric firmly in contact with the said support S.

Advantageously, as shown in Figures 2 and 3, the support 2 can be positioned (sandwiched) between the base B and the frame 3.

A make-up product 5 is then poured onto the frame 3, according to step d).

The make-up product 5 is better described later.

Advantageously, the outer frame of the frame 3, which has a thickness S1, acts as a container for the make-up product 5, thereby preventing the said product from leaking out around the edges of the frame and making the said product easier to spread.

Step e) envisages spreading the make-up product 5 over the surface 4 of the frame 3, so that the said product settles on the part of the support 2 facing the passage area P1, P2, P3 after flowing through the holes F.

The make-up product 5 is spread all over the surface 4, advantageously using a squeegee 6 or spatula, ensuring that the latter passes into the non-screened areas, therefore into the holes F in the passage area P1 (or in the passage areas P1, P2, P3).

The squeegee 6, can have a vulcanised rubber surface, which is the part that is in contact with the surface 4 and has a hardness of preferably between 65 shore and 75 shore. This way, it is possible to apply pressure to the said surface 4 without damaging the screened part S or the part featuring the holes F.

The pressure applied, help to make the make-up product to pass through the holes, reaching the support.

Subsequently, the frame 3 is removed from the support 2 in step f), and in step g) the make-up product 5 on the support is dried/desiccated; this step removes the solvent content of the make-up product 5, that becomes the make-up product 5'.

Basically, the make-up product **5'** is the make-up product 5 dried.

Preferably, the support 2 holding the make-up product 5 is dried in the oven at a temperature of 25-70°C, more preferably 30-50°C, and even more preferably at approximately 40°C for a few hours, preferably for 5-10 hours, and even more preferably for approximately 7 hours.

Advantageously, the make-up product 5 is dried until the residual moisture thereof is less than 3% of the final weight of the dried make-up product 5'.

Once dried, the make-up article 1 is obtained, which is formed of the support 2 with the make-up product 5' arranged on top thereof, the said make-up product 5' forming the decoration 10 and adhering perfectly to the support.

A method has been disclosed above for producing a decoration 10 (on the support 2) featuring a single colour.

To obtain a decoration 10 with multiple colours, it is possible to divide the decoration 10 into multiple levels, each one thereof corresponding to a desired colour. For each level, it is then possible to make a transparency so as to make a further frame for each colour of the decoration.

The steps described above are then repeated in succession with the further frames and with further make-up products in different colours until the desired final decoration is achieved.

The drying phase can be performed after using each frame (with the corresponding make-up product 5), or as a final stage, after having used all the frames (each with the correspondent make-up product 5).

The make-up article 1 is therefore made using the method described above.

Obviously, depending on the support 2, the make-up article 1 may take any shape.

For example, as shown in Figure 7, it may take the form of stickers (for example circular stickers), which can be multi-coloured.

In this case, the support S can be made of silicone-coated paper with a sticker placed on the opposite side to the side on which the decoration 10 is impressed.

To make the make-up product 5 (also referred to as the "paste" hereafter) a conventional blend of cosmetic powders is prepared, which can be prepared - for example - according to the disclosures in patent EP2189150 B1.

Further specific steps are then carried out which contribute to the formation of the make-up product 5.

In particular, the preparation process comprises the following steps:
i. supplying a blend of cosmetic powders;
ii. mixing the said cosmetic powders with an emulsion comprising water and at least one carrageenan, preferably in a mixer at a speed of 5-500 rpm, to obtain a paste, i.e. the make-up article;
in step i., the cosmetic powders are preferably supplied in the form of a blend, i.e. a homogeneous mix, by using a high-speed mixer.

Preferably, step ii. of the process is carried out in a special turboemulsifier.

Preferably, in step ii, the cosmetic powders and the emulsion are mixed in a weight ratio ranging from 10:90 to 50:50, more preferably from 10:90 to 40:60.

In preferred embodiments, the cosmetic powders and the emulsion are mixed in a weight ratio of approximately 30:70.

More preferably, in step ii., the said emulsion comprises water and:
a) 0.5-10% by weight of at least one carrageenan;
b) 0.05-5% by weight of a non-emulsifying ester of choice from isononyl isononanoate, isodecyl neopentanoate, octyldodecyl neopentanoate, isostearyl isostearate, isopropyl myristate, isocetyl stearate, and mixtures thereof;
c) 0.1-5% by weight of methyl glucose dioleate ether;
d) 0.1-5% by weight of two or more of the following: glyceryl stearate, behenyl alcohol, palmitic acid, stearic acid, lecithin, lauryl alcohol, myristyl alcohol, and cetyl alcohol;
e) 1-10% by weight of glycerine;
with respect to the weight of the emulsion.

In preferred embodiments, in step ii., the said emulsion comprises water and:
a) 0.5-5% by weight of at least one carrageenan;
b) 0.05-5% by weight of a non-emulsifying ester of choice from isononyl isononanoate, isodecyl neopentanoate, octyldodecyl neopentanoate, isostearyl isostearate, isopropyl myristate, isocetyl stearate, and mixtures thereof;
c) 0.5-3% by weight, preferably approximately 2% by weight, of a methyl glucose dioleate ether, preferably PEG-120 methyl glucose dioleate, such as Glucamate^{™} DOE-120 by Lubrizol;
d) 0.5-3% by weight, preferably approximately 2% by weight, of glyceryl stearate, behenyl alcohol, palmitic acid, stearic acid, lecithin, lauryl alcohol, myristyl alcohol, and cetyl alcohol, such as the mix produced by Ashland and marketed with the trade name ProLipid^{™} 141,
e) 1-10% by weight of glycerine;
with respect to the weight of the emulsion.

The process for preparing the make-up product 5 is preferably a process for preparing an eyeshadow wherein the cosmetic powders provided in step i. are obtained by mixing the constituents of the eyeshadow (such as: colorants, talc, mica, silica, pigments) and sieving to ensure a blend with particle homogeneity.

Preferably, the blend of cosmetic powders comprises pearlescent pigments, typically formed of mica coated with titanium oxide, in a concentration of between 20 and 70% by weight, with respect to the total weight of the blend of powders.

Reference will now be made to the make-up product 5' (which is dried) but some characteristics or types of product will be specified which are obviously also applicable for the production method thereof mentioned above.

With regards to the make-up product 5' in Figure 5, i.e. the make-up product 5 in Figure 3 after drying, the said product can comprise a blend of cosmetic powders and:
a) 0.1-15% by weight of at least one carrageenan with respect to the make-up product.

Carrageenans are a family of compounds of polysaccharide nature (CAS Number: 9000-07-1) obtained by boiling red algae from the rocky coast of the North Atlantic (Chondruscrispus and Gigartinamamitiosa, also known as Irish moss, marine lichen or carragheen).

There are mainly three types of carrageenans: kappa (k-), iota (i-) and lambda (l-), which differ from one another in the number and location of the sulphate groups and the bond that joins the individual monomers. As a rule, the greater the number of sulphate groups, the lower the viscosity capacity.

For the objects of the present invention, iota (i-) carrageenans are preferred, due to their ability to produce soft, extremely elastic gels. The addition of glucose and potassium chloride increases this characteristic.

Kappa (k-) carrageenans can also be used, as their characteristic is that they stabilise the formulation and improve the texture of the product.

Preferably, at least two different carrageenans are present, each one having a different viscosity capacity.

There are various commercial carrageenans available, each one having specific viscosities and properties, and such carrageenans can be used together in the composition of the make-up product 5'. Particularly preferable are the following carrageenans: Safic care t ck-1 marketed by Safic-alcan, iota carrageenan by Flower Tales Cosmetics, and GENUVISCO^{®} CG-131, iota carrageenan by CP Kelco.

Depending on the make-up product in question, the term "cosmetic powder blend" refers to a blend of solid powder ingredients of which such make-up products are typically constituted. For example, cosmetic powders may include ingredients such as Talc, Mica, Bismuth Chloride Oxide (CI77163), Iron Oxides (CI77491-2-9), Ultramarine Blue (CI77007), Manganese Violet (CI77742), Chromium Hydroxide (CI77289), Chromium Oxide (CI77288), Ferric Ammonium Ferrocyanide (CI77510), Titanium Dioxide (CI77891), D&C Red 7 CA Lake, D&C Red 19 Al Lake, D&C Red 6 Ba Lake, D&C Red 3 Al Lake, D&C Red 9 BA Lake, D&C Red 21 Al Lake, D&C Yellow 5 Al Lake, D&C Red 30 Al Lake, D&C Yellow 10 Al Lake, D&C Red 27 Al Lake, D&C Yellow 5 Al Lake, D&C Orange 5, FD&C Yellow 6 Al Lake, FD&C Blue 1 Al Lake, D&C red 36 Al Lake, Carmine (CI75470), Fluorphlogopite (Synthetic Mica), Boron Nitride, Calcium Aluminium Borosilicate, Magnesium Aluminium Borosilicate.

(In the list above, the "CI" number shown in brackets is the Colour Index reference number; for organic lacquers, the CTFA NAME is also included).

Preferably, the make-up product 5' comprises a blend of cosmetic powders and the following:
a) 0.1-15% by weight of at least one carrageenan with respect to the make-up product, the said make-up product furthermore having at least one of the following technical characteristics:
   - hardness [shore A]: 20 - 60 (ASTM D2240)
   - tensile strength [N/mm²]: 2 - 10 (ASTM D412)
   - elongation [%]:5-15 (ASTM D412)
   - tear resistance [N/mm]: 10 - 40 (ASTM D624).

Indeed, these technical characteristics (preferably all) ensure the product remains suitably flexible, therefore resistant to impacts and pleasant to the touch, while remaining solid and adhering perfectly to the support 2.

More preferably, the make-up product 5' comprises a blend consisting of 40-60% by weight of cosmetic powders and the following:
a) 0.5-10% by weight of at least one carrageenan;
b) 0.05-5% by weight of a non-emulsifying ester of choice from isononyl isononanoate, isodecyl neopentanoate, octyldodecyl neopentanoate, isostearyl isostearate, isopropyl myristate, isocetyl stearate, and mixtures thereof;
c) 0.1-5% by weight of methyl glucose dioleate ether;
d) 0.1-5% by weight of two or more of the following: glyceryl stearate, behenyl alcohol, palmitic acid, stearic acid, lecithin, lauryl alcohol, myristyl alcohol, and cetyl alcohol;
e) 5-10% by weight of glycerine;
with respect to the weight of the product.

In preferred embodiments, the said non-emulsifying ester b) is isononyl isononanoate.

In further preferred embodiments, the ingredient d) is a blend of glyceryl stearate, behenyl alcohol, palmitic acid, stearic acid, lecithin, lauryl alcohol, myristyl alcohol, and cetyl alcohol.

The make-up product 5' features a unique texture, which includes several characteristics usually associated with different types of products that have never previously coexisted in a single formula. For example, the said product has a compact appearance and a consistency similar to that of an extrudate when dispensed. The consistency, meanwhile, is comparable to a poured product when applied to the skin.

Advantageously, the make-up product 5' is inert when dry (i.e. the said product does not create colour or release components when placed in contact with a dry surface, including the skin).

When moistened however, i.e. placed in contact with polar substances such as water, glycols, or alcohols, the said product immediately has payoff (i.e. immediately releases part of the product, for example colour on the skin upon application).

Thanks to these unique characteristics, the make-up product 5', placed on top of the support 2, can be transported and touched without releasing colour undesirably, while one simply has to moisten the said product to render the latter usable for make-up purposes.

The present invention is therefore also aimed at the use of the said make-up article 1 to apply make-up to the skin by means of a method comprising the following steps in series:
- moistening the make-up product 5' placed on top of the support 2 with a polar substance, preferably water, and
- applying the make-up substance released by the thus moistened product to the skin.

When applied to the skin, the make-up product 1 forms a perfectly adherent but imperceptible film of make-up on the skin, which also has a long hold.

From a further perspective, the make-up product 5' placed on the support 2 can have a height ranging from between 0.2 and 1 mm, preferably between 0.2 and 0.8 mm, therefore preferably equal to the diameter of the strands that determine the surface 4.

Unlike the compact products typical of the prior art, the make-up article 1 according to the present invention, thus obtained, can be directly placed in a pouch made of a flexible material, such as paper or silicone, without needing to be placed in a rigid godet to prevent breakage, as this product is flexible and resistant to impacts. It can therefore be handled directly, without the need for protective wrappers or containers.

To the touch, the make-up product 5' seems solid, almost rubbery, and dry, but extremely flexible and resistant at the same time.

A make-up product 5' such as the one described is particularly flexible. Therefore, it is particularly suitable to be coupled to paper supports 2, which are also very flexible (see Figure 7).

In practice, the make-up article 1 can be bent into what is essentially a U shape without the make-up product 5' breaking, detaching from the support 2, or cracking on the surface thereof.

Furthermore, the make-up product 5' has a high resistance to thermal shock and especially to heat, unlike poured or cream products.

A further advantage lies in the fact that the make-up product 5' does not release colour unless moistened with a polar solvent, for example with water, or glycols, preferably at least of the pentylene type.

Using water, the product dries on the skin in a very short space of time (from 1 to 3 minutes).

Various embodiments of the innovation have been disclosed herein, but further embodiments may also be conceived using the same innovative concept.

## Claims

1. Method for producing a make-up article (1) comprising the steps of:
a. preparing a non-making up support (2);
b. arranging a frame (3) having a surface (4) formed by a screened area (S) and at least one passage area (P1, P2, P3) defining the shape of a decoration to be impressed on the support, the passage area (P1, P2, P3) being formed by a plurality of holes (F) mutually placed side by side, the screened area (S) being that part of the surface (4) free from holes (F), the surface (4) being a fabric and each hole (F) being perimetrically bounded by threads defining the weft and a warp of the fabric;
c. placing the frame (3) over the support (2);
d. pouring the make-up product (5) onto the frame;
e. spread the make-up product (5) on the surface (4), so that it deposits on the part of the support facing the passage area (P1, P2; P3)
f. removing the frame (3) from the support;
g. allowing the make-up remover (5) to dry over the support (2).

2. Method according to claim 1, wherein the support (2) is one of the following materials: paper, cardboard, plasticized or waterproofed paper, fabric, plastic.

3. Method according to claim 1, wherein each hole (F) has an area between 0.01 and 0.12 mm², preferably between 0.015 and 0.12 mm².

4. Method according to claim 1, wherein each hole (F) has, in plan view, a quadrangular cross-section, preferably a square cross-section.

5. Method according to claim 4, wherein the screened area (S) is defined by said fabric impregnated in a hardened water-soluble photosensitive emulsion.

6. Method according to claim 1, wherein, after step g:) h. a further frame is provided substantially identical to the frame of step b), but with a different conformation of the passage area (P1, P2, P3) that defines a further passage area;
i. the further frame is arranged over the support (S);
h. a further make-up product of a different color from that of step d) is poured over the further frame;
i. the further make-up product is spread on the surface (4), so that it deposits on the part of the support facing the further passage area;
j. the further frame is removed from the support;
k. the make-up product (5) is allowed to dry on top of the support (2).

7. Method according to claim 6, wherein the steps h)-k) are repeated with as many frames and as many different colored make-up products as are necessary to obtain the desired final decoration on the support (S).

8. Method according to claim 2, wherein the support (S) is an adhesive.

9. Method according to claim 1, wherein the make-up product (5) comprises a mixture of cosmetic powders and:
(a) 0.1-15% by weight of at least one carrageenan, on the weight of the cosmetic product.

10. Method according to claim 9, wherein said at least one carrageenan is carrageenan-iota.

11. Method according to claim 9, wherein the makeup product (5) comprises 40-60% by weight of cosmetic powder mixture and:
(a) 0.5-10% by weight of at least one carrageenan;
(b) 0,05-5% by weight of a non-emulsifying ester chosen from isononyl isonanoate, isodecylneopentanoate, octyldodecylneopentanoate, isostearylisostearate, isopropyl myristate, isocetyl stearate, and mixtures thereof;
(c) 0.1-5% by weight of methyl glucose ether dioleate;
(d) 0.1-5% by weight of two or more of Glyceryl Stearate, Behenyl Alcohol, Palmitic Acid, Stearic Acid, Lecithin, Lauryl Alcohol, Myristyl Alcohol, and CetylAlcohol;
(e) 5-10% by weight of glycerine;
by weight of the product.

12. Make-up article (1) made by the method of claim 1.
